# EUROPÄISCHE PATENTSCHRIFT

(11) **EP 2 462 349 B1**
(45) Veröffentlichungstag und Bekanntmachung des Hinweises auf die Patenterteilung: **10.05.2017**
(21) Anmeldenummer: 11717462.3
(22) Anmeldetag: 04.03.2011
(51) Int. Cl.: A61F 9/007, F04C 15/00, F04D 27/02, F04B 49/00

(54) **VORRICHTUNG ZUR DRUCKLUFTERZEUGUNG FÜR EIN MEDIZINISCHES HANDGERÄT**
DEVICE FOR GENERATING COMPRESSED AIR FOR A MEDICAL HAND-DEVICE
DISPOSITIF GÉNÉRATEUR D'AIR COMPRIMÉ POUR UN INSTRUMENT À MAIN MÉDICAL

(30) Priorität: 05.03.2010 DE 102010010555
(43) Veröffentlichungstag der Anmeldung: 13.06.2012
(73) Patentinhaber: Geuder AG, 69126 Heidelberg (DE)
(72) Erfinder: GEUDER, Volker, 69126 Heidelberg (DE); FRAUENFELD, Dieter, 69121 Heidelberg (DE); BECHTEL, Helmut, 69121 Heidelberg (DE)
(74) Vertreter: Patent- und Rechtsanwälte Ullrich & Naumann
(86) Internationale Anmeldenummer: PCT/DE2011/000225
(87) Internationale Veröffentlichungsnummer: WO 2011/107086

(56) Entgegenhaltungen:
- EP-A1- 0 431 287
- DE-A1-102006 034 179
- DE-C- 916 203
- US-A1- 2005 123 407
- US-A1- 2009 204 022
- US-B1- 6 383 203

## Beschreibung

Die vorliegende Erfindung betrifft eine Vorrichtung zur Drucklufterzeugung zum Antrieb mindestens eines medizinischen Handgeräts, Handstücks oder Handgriffs, insbesondere eines chirurgischen Handgeräts zur Durchführung ophthalmologischer Operationen, beispielsweise eines Cutter.

Vorrichtungen der hier in Rede stehenden Art dienen zum Druckluftantrieb bestimmter medizinischer Handgeräte und werden entweder als unabhängige Bauteile oder beispielsweise in kompakten Steuer-/Versorgungseinheiten integriert dem Operateur zur Verfügung gestellt, wobei es auf die Versorgung mit gleichförmiger oder pulsierender Druckluft ankommt.

Steuer-/Versorgungseinheiten, die solche Vorrichtungen zur Drucklufterzeugung beinhalten, sind als bauliche Einheit zu verstehen und versorgen beispielsweise in der Ophthalmologie die vom Operateur benötigten Handgeräte mit Druckluft, pulsierender Druckluft, Unterdruck, Licht, Strom, Flüssigkeit, etc. und dienen ebenso zum Erzeugen eines Unterdrucks zum Absaugen von Flüssigkeit, beispielsweise im Rahmen der Entfernung des Linsenkörpers nach dessen Zertrümmerung, wobei dabei die Linsentrümmer gemeinsam mit einer Spülflüssigkeit abgesaugt wird.

Bei den durch die Steuer-/Versorgungseinheit zu betreibenden medizinischen Handgeräten kann es sich um Handgeräte unterschiedlichster Art handeln, beispielsweise um augenchirurgisches Instrumente zum Zertrümmern von Linsen (Cutter) und zum Absaugen von Linsentrümmern aus dem Auge. Insoweit sei lediglich beispielhaft auf die DE 40 08 594 C2 verwiesen. In diesem Falle wird das Handgerät von der Steuer-/Versorgungseinheit her mit pulsierender Druckluft und Spülflüssigkeit versorgt, wobei die zertrümmerten Linsenteile per Unterdruck abgesaugt werden. Die Schlauchverbindung zwischen dem Handgerät und der Steuer-/Versorgungseinheit dient dabei sowohl zur Versorgung als auch zur Entsorgung.

Grundsätzlich gibt es zwei Möglichkeiten, dem Operateur Druckluft bzw. pulsierende Druckluft zur Verfügung zu stellen, nämlich entweder über eine zentrale Druckluftversorgung, sozusagen per Wandanschluss, oder entsprechend den voranstehenden Ausführungen über eine Steuer-/Versorgungseinheit, die eine entsprechende Vorrichtung als integrale Baugruppe umfasst. Dies ist in der Praxis bereits der Fall, wobei es der Operateur regelmäßig als störend empfindet, wenn der dort vorgesehenen Kompressoren mit relativ hoher Förderleistung arbeitet, nämlich aufgrund der damit verbundenen Geräuschentwicklung. Des Weiteren besteht ein Problem dahingehend, dass eine Regelung des Kompressors allenfalls bedingt stattfindet, jedoch keineswegs leistungsoptimiert. Schließlich erfordert die Vorkehrung eines den Anforderungen bei einer Augenoperation gerecht werdenden Kompressors einen erheblichen Bauraum in der Steuer-/Versorgungseinheit, was dem ständigen Streben auf Reduktion des durch die Steuer-/Versorgungseinheit beanspruchten Bauraums entgegensteht.

Der vorliegenden Erfindung liegt daher die Aufgabe zugrunde, eine Vorrichtung zur Drucklufterzeugung zum Antrieb mindestens eines medizinischen Handgeräts, Handstücks oder Handgriffs, insbesondere eines chirurgischen Handgeräts zur Durchführung ophthalmologischer Operationen, derart auszugestalten und weiterzubilden, dass sie bei leistungsoptimiertem Betrieb geräuscharm arbeitet und möglichst klein baut. Aus dem Stand der Technik, wie zum Beispiel US 2005/0123407 A1, sind Systeme mit den Kompressoren nachgeordneten Schalldämpfern bekannt. Die voranstehende Aufgabe ist durch die Merkmale des Patentanspruchs 1 gelöst. Danach ist die hier in Rede stehende Vorrichtung, die beispielsweise zur Druckluftbeaufschlagung eines Cutters dient, mit zwei asynchron geschalteten Kompressoren ausgestattet, deren Betrieb zur Leistungsoptimierung auf ganz besondere Art und Weise geregelt ist. Die Vorrichtung ist geeignet, in eine Steuer-/Versorgungseinheit zum Versorgen entsprechender chirurgischer Handgeräte integriert zu werden.

In ganz besonders vorteilhafter Weise arbeiten die beiden Kompressoren nicht etwa gleichberechtigt nebeneinander mit identischer Ansteuerung, lassen sich vielmehr unabhängig voneinander ansteuern. Im Konkreten ist es denkbar, dass der erste Kompressor bis zu einer vorgebbaren Leistung alleine arbeitet und bei einer darüber liegenden Leistungsanforderung der zweite Kompressor automatisch zugeschaltet wird. Des Weiteren ist es möglich, bei einer geringen Leistungsanforderung nur mit einem kleinen Kompressor bei geringer Geräuschentwicklung und kleiner Wärmeentwicklung solange zu arbeiten, bis der eine Kompressor der Leistungsanforderung nicht mehr gerecht wird. Es ist im Konkreten denkbar, dass zum Antrieb eines Cutters der zweite Kompressor ab einer vorgebbaren Schnittzahl pro Zeiteinheit, beispielsweise ab 800 Schnitte pro Minute, zugeschaltet wird, so dass eine höhere Leistung bzw. ein höherer Volumenstrom an Druckluft geliefert werden kann, nämlich um über 800 Schnitte pro Minute zu generieren. Beliebige andere Kombinationen von Leistungsstufen sind denkbar, entsprechend der Auslegung der beiden Kompressoren.

Auch ist es denkbar, dass beide Kompressoren zunächst mit einer Minimalleistung gemeinsam arbeiten, nämlich gemeinsam mit geringer Geräuschentwicklung und geringer Wärmeemission. Entsprechend der Leistungsanforderung wird der eine Kompressor hochgefahren, nämlich solange, bis er die insgesamt erforderliche Leistung bzw. den angeforderten Volumenstrom an Druckluft nicht mehr liefern kann. Erst dann, oder wenn das Hinzuschalten des zweiten Kompressors aus Gründen der Leistungsoptimierung sinnvoll erscheint, wird die Leistung des zweiten Kompressors - allmählich - erhöht, nämlich wenn eine entsprechende Gesamtleistung beider Kompressoren angefordert wird. Entsprechend den voranstehenden Ausführungen wird auf einen optimalen Betrieb eines jeden der beiden Kompressoren geachtet, so dass insgesamt ein leistungsoptimierter Betrieb beider Kompressoren unter Berücksichtigung einer geringstmöglichen Lärm- und Wärmeemission möglich ist.

An dieser Stelle sei angemerkt, dass ein einziger großer Kompressor mit entsprechendem Leistungsbild wesentlich größer baut als zwei kleiner Kompressoren, die sich in der Leistung ergänzen. Dies gilt insbesondere für Gleichstromkompressoren, beispielsweise für Kolbenverdichter.

Grundsätzlich ist es denkbar, dass beide Kompressoren identisch ausgeführt sind. Je nach Leistungsanforderung, d.h. unter Berücksichtigung der an die Steuer-/Versorgungseinheit anzuschließenden Handgeräte, kann es von Vorteil sein, wenn beide Kompressoren unterschiedliche Förder-/Verdichtungsleistungen haben, nämlich dann, wenn angestrebt wird, mit dem ersten Kompressor ein möglichst großes Spektrum aller Bearbeitungsvarianten abzudecken. So könnten die Kompressoren derart ausgelegt sein, dass ein Hinzuschalten des zweiten Kompressors selten erfolgt, nämlich unter Berücksichtigung üblicher Anforderungen.

Auch ist es denkbar, dass beide Kompressoren unterschiedlicher Bauart sind, was impliziert, dass die Förder-/Verdichtungsleistungen sich ebenfalls voneinander unterscheiden. Wie bereits zuvor erwähnt, kann es sich bei den Kompressoren um mit Gleichstrom betriebene Kompressoren handeln, beispielsweise um Kolbenverdichter. Andere geeignete Antriebe sind denkbar.

Zum Betrieb der beiden Kompressoren ist in vorteilhafter Weise eine besondere Treiberstufe vorgesehen, nämlich eine asynchrone Treiberstufe, die regelmäßig über Verstärker gespeist wird. Über die Treiberstufe werden die beiden Kompressoren asynchron angesteuert, wobei die von den Kompressoren erzeugte Druckluft über ein 3/2 Luftventil und einen sich daran anschließenden Druckluftschlauch an das Handstück, beispielsweise den Cutter, geführt wird. Während der ersten Kompressor beispielsweise eine Schnittzahl von 0 bis 800 Schnitte pro Minute ermöglicht, lassen sich bei Hinzuschalten des zweiten Kompressors Luftdrücke erzielen, die eine Leistung für Schnitte über 800 Schnitt pro Minute ermöglichen.

Schließlich ist es von Vorteil, wenn dem Benutzer, insbesondere dem Operateur, eine für ihn nachvollziehbare Einstellmöglichkeit der beiden Kompressoren an die Hand gegeben wird, wobei es den Operateur wenig interessiert, mit welcher tatsächlichen Leistung der eine und/oder der andere Kompressor arbeitet. Für den Operateur ist beispielsweise beim Anschluss eines Cutters von Bedeutung, mit welcher Schnittleistung pro Minute der Cutter arbeiten kann. So bietet es sich an, dass die beiden Kompressoren über eine grafische Benutzeroberfläche in Bezug auf ihre Zuschaltung und Leistung unabhängig voneinander einstellbar sind, beispielsweise dahingehend, dass man in Bezug auf den ersten Kompressor eine Bandbreite möglicher Einstellungen erkennt und dabei definiert, ab welcher Schnittzahl der zweite Kompressor hinzugeschaltet wird. Ebenso ist es denkbar, dass die Steuerung über einen geeigneten Mikrocontroller bzw. über einen Prozessor bei einer vom Operateur geforderte Schnittzahl pro Minute eine optimierte Steuerung der beiden Kompressoren unter Berücksichtigung der diesbezüglichen Leistungsdaten automatisch vornimmt, nämlich unter Berücksichtigung geringstmöglicher Lärm- und Wärmeemissionen sowie unter Berücksichtigung eines die beiden Kompressoren schonenden Betriebs. Beliebige Optimierungsansätze sind realisierbar.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht die grundsätzliche Anordnung einer erfindungsgemäßen Vorrichtung umfassenden Steuer-/Versorgungseinheit, an die über einen Druckluftschlauch ein Cutter angeschlossen ist und
- Fig. 2: in einem schematischen Blockschaltbild eine grundsätzliche Möglichkeit zur Kompressorsteuerung im Rahmen der erfindungsgemäßen Vorrichtung.

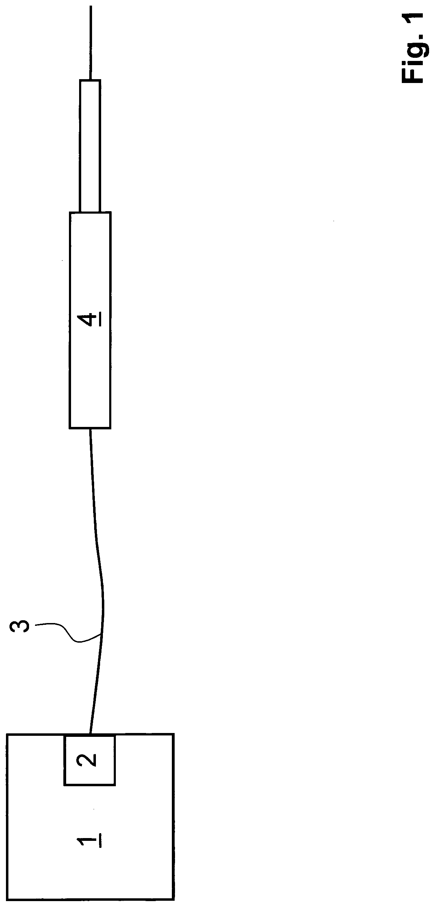

Fig. 1 zeigt in einer schematischen Darstellung einer Steuer-/Versorgungseinheit 1, die eine erfindungsgemäße Vorrichtung 2 als integrale Baugruppe umfasst. Insoweit ist die erfindungsgemäße Vorrichtung 2 dort lediglich symbolisch angedeutet. Die Steuer-/Versorgungseinheit 1 umfasst einen Druckluftanschluss, so dass die über die Vorrichtung 2 erzeugte Druckluft über eine Druckluftleitung 3 zu einem chirurgischen Handgerät gelangt, wobei es sich dabei im Konkreten um einen Cutter 4 handelt.

Die vom Operateur benötigte Druckluftleistung kann der Operateur üblicherweise über einen Fußschalter anfordern, der mit der Steuer-/Versorgungseinheit 1 verbunden ist. Da es auf die diesbezügliche Betätigung im Rahmen der erfindungsgemäßen Lehre nicht ankommt, wird auf eine Darstellung eines solchen Fußschalters verzichtet. Weitere Ausführungen dazu sind hier nicht erforderlich.

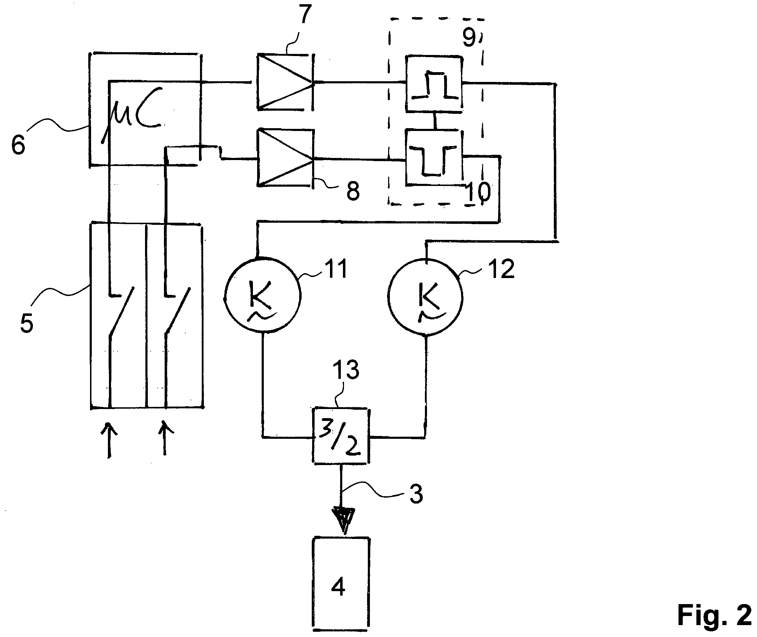

Fig. 2 zeigt in einem schematischen Blockschaltbild eine mögliche Kompressorsteuerung zur Realisierung der erfindungsgemäßen Vorrichtung, wobei die Einstellung über eine grafische Benutzeroberfläche 5 (GUI) erfolgen kann. Grundsätzlich ist es denkbar, dass die Steuer-/Versorgungseinheit 1 ein Touchscreen umfasst, welches zur Einstellung dient.

Entsprechend der über die grafische Benutzeroberfläche 5 gewählten Einstellung wird über einen Mikrocontroller 6 und über Verstärker 7, 8 eine asynchrone Treiberstufe 9, 10 beaufschlagt, die wiederum die beiden Kompressoren 11, 12 ansteuert. Ein Betrieb der beiden Kompressoren 11, 12 ist entsprechend den voranstehenden Ausführungen aus der allgemeinen Beschreibung möglich. Die dort erzeugte Druckluft - durch den Betrieb eines einzigen Kompressors oder bei kombiniertem Betrieb beider Kompressoren - gelangt über ein 3/2 Luftventil 13 und über die Druckluftleitung 3 zum Cutter 4, nämlich entsprechend der Einstellung über die Benutzeroberfläche 5 und unter Berücksichtigung der tatsächlich über einen Fußschalter oder dgl. angeforderten Leistung.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich der beispielhaften Erörterung der beanspruchten Lehre dient, diese jedoch nicht auf das Ausführungsbeispiel einschränkt.

### Bezugszeichenliste

- 1: Steuer-/Versorgungseinheit
- 2: Vorrichtung zur Drucklufterzeugung
- 3: Druckluftleitung
- 4: Cutter
- 5: grafische Benutzeroberfläche (GUI)
- 6: Mikrocontroller
- 7: Verstärker
- 8: Verstärker
- 9: asynchrone Treiberstufe
- 10: asynchrone Treiberstufe
- 11: Kompressor
- 12: Kompressor
- 10: Mikrocontroller
- 13: Luftventil

## Patentansprüche

1. Vorrichtung zur Drucklufterzeugung zum Antrieb mindestens eines medizinischen Handgeräts, Handstücks oder Handgriffs, insbesondere eines chirurgischen Handgeräts zur Durchführung ophthalmologischer Operationen, beispielsweise eines Cutter, vorzugsweise integriert in eine Steuer-/Versorgungseinheit, mit zwei asynchron geschalteten Kompressoren, deren Betrieb zur Leistungsoptimierung geregelt ist,
**dadurch gekennzeichnet, dass** die in den Kompressoren erzeugte Druckluft über ein 3/2 Luftventil über eine Druckluftleitung zum Handstück geleitet wird.

2. Vorrichtung nach Anspruch 1, **dadurch gekennzeichnet, dass** der erste Kompressor (11) bis zu einer vorgebbaren Leistung alleine arbeitet und bei einer darüber liegenden Leistungsanforderung der zweite Kompressor (12) automatisch zugeschaltet wird.

3. Vorrichtung nach Anspruch 1 oder 2, **dadurch gekennzeichnet, dass** zum Antrieb eines Cutter (4) der zweite Kompressor (12) ab einer vorgebbaren Schnittzahl pro Zeiteinheit, vorzugsweise ab 800 Schnitte pro Minute, zugeschaltet wird.

4. Vorrichtung nach einem der Ansprüche 1 bis 3, **dadurch gekennzeichnet, dass** beide Kompressoren (11, 12) zunächst mit einer Minimalleistung gemeinsam arbeiten und die Leistung des zweiten Kompressors (12) erst dann erhöht wird, wenn eine entsprechende Gesamtleistung angefordert wird.

5. Vorrichtung nach einem der Ansprüche 1 bis 4, **dadurch gekennzeichnet, dass** beide Kompressoren (11, 12) identisch sind.

6. Vorrichtung nach einem der Ansprüche 1 bis 4, **dadurch gekennzeichnet, dass** beide Kompressoren (11, 12) unterschiedliche Förder-/Verdichtungsleistung haben.

7. Vorrichtung nach einem der Ansprüche 1 bis 4, **dadurch gekennzeichnet, dass** beide Kompressoren (11, 12) unterschiedlicher Bauart sind.

8. Vorrichtung nach einem der Ansprüche 1 bis 7, **dadurch gekennzeichnet, dass** die Kompressoren (11, 12) mit Gleichstrom arbeiten.

9. Vorrichtung nach einem der Ansprüche 1 bis 8, **dadurch gekennzeichnet, dass** die Kompressoren (11, 12) als Kolbenverdichter ausgeführt sind.

10. Vorrichtung nach einem der Ansprüche 1 bis 9, **dadurch gekennzeichnet, dass** die Kompressoren (11, 12) über eine asynchrone Treiberstufe geschaltet sind.

11. Vorrichtung nach einem der Ansprüche 1 bis 10, **dadurch gekennzeichnet, dass** die in den Kompressoren (11, 12) erzeugte Druckluft über ein mit vorgebbarer Frequenz geschaltetes Proportionalventil über eine Druckluftleitung (3) zum Handstück geleitet wird.

12. Vorrichtung nach einem der Ansprüche 1 bis 11, **dadurch gekennzeichnet, dass** die beiden Kompressoren (11, 12) vorzugsweise über eine grafische Benutzeroberfläche in Bezug auf ihre Zuschaltung und Leistung unabhängig voneinander einstellbar sind.

## Claims

1. Apparatus for generating compressed air for driving at least one medical hand-held device, handpiece or handle, in particular a surgical hand-held device for performing ophthalmological operations, for example a cutter, preferably integrated in a control or supply unit, comprising two asynchronously connected compressors, the operation of which is controlled to optimise performance, **characterised in that** the compressed air generated in the compressors is directed to the handpiece through a compressed air pipe via a 3/2 air valve.

2. Apparatus according to claim 1, **characterised in that** the first compressor (11) operates independently up to a predeterminable output and the second compressor (12) is automatically connected when the output requirement exceeds said predeterminable output.

3. Apparatus according to either claim 1 or claim 2, **characterised in that**, in order to drive a cutter (4), the second compressor (12) is connected once a predeterminable number of cuts per time unit has been reached, preferably 800 cuts per minute.

4. Apparatus according to any one of claims 1 to 3, **characterised in that** the two compressors (11, 12) first operate together with a minimum output and the output of the second compressor (12) is only increased when a corresponding total output is required.

5. Apparatus according to anyone of claims 1 to 4, **characterised in that** the two compressors (11, 12) are identical.

6. Apparatus according to any one of claims 1 to 4, **characterised in that** the two compressors (11, 12) have different conveying/compression outputs.

7. Apparatus according to any one of claims 1 to 4, **characterised in that** the two compressors (11, 12) are constructed differently.

8. Apparatus according to any one of claims 1 to 7, **characterised in that** the compressors (11, 12) operate using DC current.

9. Apparatus according to any one of claims 1 to 8, **characterised in that** the compressors (11, 12) are designed as piston compressors.

10. Apparatus according to any one of claims 1 to 9, **characterised in that** the compressors (11, 12) are connected via an asynchronous driver stage.

11. Apparatus according to any one of claims 1 to 10, **characterised in that** the compressed air generated in the compressors (11, 12) is directed to the handpiece through a compressed air pipe (3) via a proportional valve switched at a predeterminable frequency.

12. Apparatus according to any one of claims 1 to 11, **characterised in that** the two compressors (11, 12) can be adjusted independently of one another with regard to the connection and output thereof, preferably via a graphical user interface.

## Revendications

1. Dispositif destiné à la production d'air comprimé pour l'entraînement d'au moins un appareil manuel, d'une pièce manuelle ou d'une poignée médicale(e), en particulier d'un appareil manuel chirurgical, pour la réalisation d'opérations ophtalmologiques, par exemple d'un cutter, de préférence intégré dans une unité de commande/d'alimentation, avec deux compresseurs montés de façon asynchrone dont le fonctionnement est régulé en vue de l'optimisation de puissance,
**caractérisé en ce que** l'air comprimé produit dans les compresseurs est conduit à la pièce manuelle par le biais d'une vanne pneumatique 3/2 voies via une conduite d'air comprimé.

2. Dispositif selon la revendication 1, **caractérisé en ce que** le premier compresseur (11) fonctionne seul jusqu'à une puissance pouvant être prédéfinie, et le deuxième compresseur (12) est mis en marche automatiquement en présence d'une demande de puissance supérieure.

3. Dispositif selon la revendication 1 ou 2, **caractérisé en ce que**, pour l'entraînement d'un cutter (4), le deuxième compresseur (12) est mis en marche à partir d'un nombre de coupes pouvant être défini par unité de temps, de préférence à partir de 800 coupes par minute.

4. Dispositif selon l'une des revendications 1 à 3, **caractérisé en ce que** les deux compresseurs (11, 12) fonctionnent d'abord conjointement avec une puissance minimale, et la puissance du deuxième compresseur (12) n'est augmentée que quand une puissance totale correspondante est demandée.

5. Dispositif selon l'une des revendications 1 à 4, **caractérisé en ce que** les deux compresseurs (11, 12) sont identiques.

6. Dispositif selon l'une des revendications 1 à 4, **caractérisé en ce que** les deux compresseurs (11, 12) ont une puissance de refoulement/compression différente.

7. Dispositif selon l'une des revendications 1 à 4, **caractérisé en ce que** les deux compresseurs (11, 12) sont de construction différente.

8. Dispositif selon l'une des revendications 1 à 7, **caractérisé en ce que** les compresseurs (11, 12) fonctionnent avec du courant continu.

9. Dispositif selon l'une des revendications 1 à 8, **caractérisé en ce que** les compresseurs (11, 12) sont réalisés en tant que compresseurs à piston.

10. Dispositif selon l'une des revendications 1 à 9, **caractérisé en ce que** les compresseurs (11, 12) sont montés par le biais d'un étage d'attaque asynchrone.

11. Dispositif selon l'une des revendications 1 à 10, **caractérisé en ce que** l'air comprimé produit dans les compresseurs (11, 12) est conduit à la pièce manuelle via une conduite d'air comprimé (3) par le biais d'une vanne proportionnelle montée avec une fréquence pouvant être prédéfinie.

12. Dispositif selon l'une des revendications 1 à 11, **caractérisé en ce que** les deux compresseurs (11, 12) peuvent être réglés indépendamment l'un de l'autre de préférence par le biais d'une interface utilisateur graphique en ce qui concerne leur mise en marche et leur puissance.
